# EUROPEAN PATENT APPLICATION

(11) **EP 4 230 720 A1**
(43) Date of publication of application: **23.08.2023**
(21) Application number: 21882215.3
(22) Date of filing: 06.10.2021
(51) Int. Cl.: C12N 1/14, A01N 63/30, C05F 11/08, A01H 15/00, A01N 65/00

(54) **STRAIN OF RUTSTROEMIA CALOPUS, COMPOSITIONS AND USES**

(30) Priority: 19.10.2020 ES 202031055
(71) Applicant: Universidad de Almeria, 04120 La Cañada de San Urbano (Almeria) (ES)
(72) Inventor: SANTOS HERNÁNDEZ, Milagrosa, 04120 La Cañada de San Urbano (ES); DIÁNEZ MARTÍNEZ, Fernando José, 04120 La Cañada de San Urbano (ES)
(74) Representative: Pons
(86) International application number: PCT/ES2021/070730
(87) International publication number: WO 2022/084564

(57) **Abstract**

The present invention describes a strain of the species *Rutstroemia calopus* that is able to promote and enhance crop growth and development, even under conditions of water or salt stress, and uses thereof. A biofertilizer composition comprising said strain and a method for enhancing plant growth are also described.

## Description

This invention is comprised in the technical sector of agricultural microbiology, more specifically in the sector relating to the application of microorganisms on agricultural crops for biofertilization. The present invention relates to a strain of the species *Rutstroemia calopus* that is able to promote and enhance crop growth and development, even under conditions of water or salt stress.

### BACKGROUND OF THE INVENTION

One of the greatest challenges in agriculture lies in reducing the risks and negative impacts of phytosanitary products and fertilizers on human health and the environment, while maintaining or improving the productivity and profitability of agricultural activity at the same time. This is only possible by means of the use of new technologies comprised in integrated production systems which reduce waste and minimise its environmental impact, avoiding the use of chemical pesticides and fertilizers.

There are many beneficial microorganisms, and use thereof in agricultural systems can improve nutrition, resistance to abiotic and biotic stresses, and provide an effective transition from production systems based on the use of chemical products to others that are more sustainable.

The mechanism of biostimulation by microorganisms improves root and shoot systems as it releases auxins, small peptides, volatile substances, and other active metabolites into the rhizosphere, promoting root branching and nutrient absorption, thereby increasing plant growth and yield.

There are various microorganisms which are associated with plants and perform important functions in plant growth and health. Said microorganisms are involved in nutrient uptake and/or hormone stimulation, and some suppress plant pathogens.

Various mechanisms, which are often indirectly related to plant growth, are involved in the suppression of plant pathogens. The document by Jumpponen A., et al, 1998. New Phytologist 140(2):295-310*),* describes brown- or black-coloured pigmented fungi associated with plant roots, *Mycelium radicus astrovirens* (MRA), which coexisted with mycorrhizal fungi and were therefore named "pseudomycorrhizal" fungi. These fungi are now called Dark Septate Endophytes (DSE) *(Rodriguez et al., 2009).*

While members of the bacterial genera *Azospirillum* and *Rhizobium* promote plant growth, *Bacillus, Pseudomonas, Serratia, Stenotrophomonas* and *Streptomyces* and the fungal genera *Ampelomyces, Coniothyrium,* and *Trichoderma* are model organisms to demonstrate their influence on plant health *(*Berg, G. 2009. Appl Microbiol Biotechnol 84, 11-18*).*

Due to these beneficial interactions between plants and microorganisms, and depending on their mode of action and effects, these products can be used as biofertilisers, plant enhancers, phytostimulants, and biopesticides, that are applied to different crops for the purpose of improving growth, development, and adaptation to abiotic stress. They are safe for human beings, livestock, crops, and the environment and are able to colonise plant roots without apparent adverse reactions. Both solid and liquid formulations can be used to produce suitable amounts of active and viable inoculums from the time the formulation is produced until it is used in the field.

The market for microorganism-based agricultural products is experiencing an annual growth rate of about 10%. The use of microorganisms and the exploitation of beneficial plant-microbe interactions offer promising and environmentally friendly strategies for conventional and organic agriculture worldwide.

Therefore, there is a need to find efficient microbiological mechanisms and agents for agriculture and alternatives to chemical control that: promote plant growth, increase field productivity, control agricultural pests and diseases, and reduce environmental and health risks without endangering human health.

### DESCRIPTION OF THE INVENTION

The inventors have identified a microorganism of the species *Rutstroemia calopus* that is able to efficiently promote crop growth, where this is the first time that a microorganism of said species has been described in the state of the art as a plant growth biostimulating or promoting agent in plants, contributing to a reduction in the use of phytosanitary chemical compounds, reducing environmental and health risk. Furthermore, the inventors have observed that said microorganism is able to promote plant growth under the conditions of water and salt stress.

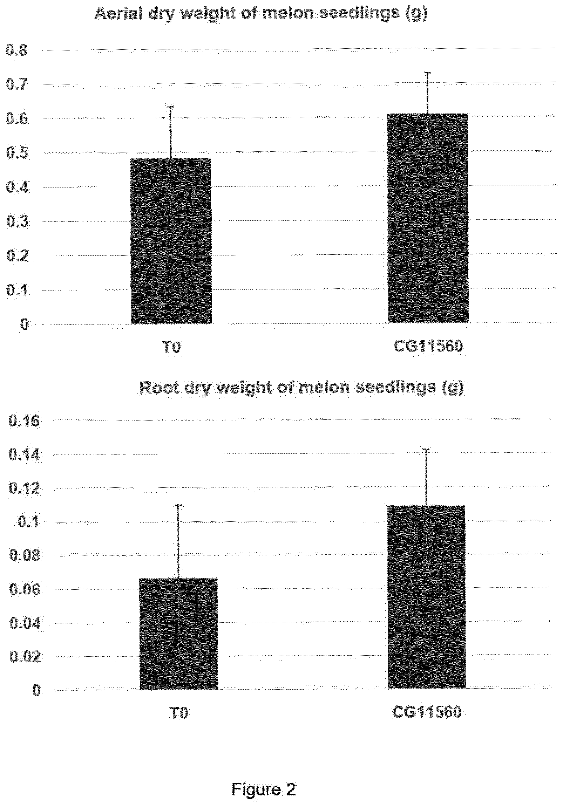

The inventors have isolated the *Rutstroemia calopus* CG11560 strain (deposit number CECT 21150) from the roots of wild plants in the coast of Almería (Spain). The inventors then carried out tests under seedbed and field conditions with melon or cucumber, without disinfecting or sterilizing the cultivation soils or substrates, as well as under conditions of salt stress. The results revealed that plant growth (dry weight of aerial and root parts and/or height of seedlings) was enhanced after the application of the *Rutstroemia calopus* CG11560 strain, with and without salt stress (Figures 2 to 8).

Therefore, in a first aspect, the invention relates to a strain of the species *Rutstroemia calopus* deposited in the Spanish Type Culture Collection (CECT) with accession number CECT 21150, hereinafter the "strain of the invention". This strain if able to stimulate, enhance, and promote plant growth.

The *Rutstroemia calopus* CG11560 strain was isolated from the roots of wild plants in the coast of Almería (Spain). The strain was deposited on 17 July 2019 under the Budapest Treaty in the Spanish Type Culture Collection as the International Depository Authority (main office at Edificio 3 CUE, Parc Científic Universitat de Valencia, C/ Catedrático Agustín Escardino, 9, 46980 Paterna (Valencia), SPAIN). The deposit number assigned was CECT 21150. The depositor of the strain was Universidad de Almería.

The *Rutstroemia calopus* CG11560 strain with deposit number CECT 21150 is a fungus that belongs to the family *Rutstroemiaceae* and the genus *Rutstroemia.* It is a poorly characterised species typical of dune areas associated with other plants such as endophytes. The inventors have observed that this new strain is able to promote or stimulate plant growth, even under conditions of salt stress.

The term "stimulating plant growth", "enhancing plant growth" used interchangeably throughout the present invention, refers to the capacity to stimulate, promote, facilitate, enhance, or aid in plant development or growth, i.e., to increase the growth or development of the root system of plants, the elongation of stems, flowering, and/or the development of fruits and seeds.

In the present invention, those microorganisms which are derived from the strain of the invention and retain the capacity to enhance or promote plant growth are also contemplated. Examples of strains or microorganisms derived from the strain of invention can be mutants having variations in their genome with respect to the genome of the strain of the invention which, however, do not affect the capacity of the strain to enhance or promote plant growth. In that sense, mutant strains derived from the strain of the invention which retain the capacity to enhance or promote plant growth are also contemplated in the present invention. Thus, in another aspect, the present invention also relates to a strain derived from the strain of the invention with the capacity to enhance or promote plant growth. The terms "mutant" or "derivative", used interchangeably herein, refers to any microorganism resulting from a mutation or modification in the DNA of an organism which leads to a character (phenotype) that is not found in the wild type strain (wt) and maintains the characteristics of the wild type strain in relation to the capacity to biologically stimulate plant growth.

The strain derived from the strain of the invention can be produced naturally or intentionally by means of mutagenesis methods known in the prior art such as, for example, without being limited to, growing the strain of the invention in the presence of mutagenic or stress-causing agents, or by means of genetic engineering aimed at modifying specific genes. In that sense, genetically modified mutants derived from the strain of the invention that retain their capacity to enhance or improve plant growth are also contemplated in the present invention.

In another preferred embodiment, the strain of the invention can be in the form of viable cells. The term "viable cells" used in the present invention refers to living or viable cells which, when supplemented with or incubated in a suitable medium, are able to divide and reproduce.

The viable cells of the strain of the invention can be in the form of mycelium or microsclerotia. Therefore, in a particular embodiment, the viable cells of the strain of the invention are in the form of microsclerotia.

Another aspect of the present invention relates to a biologically pure culture of the strain of the invention, hereinafter the "biologically pure culture of the invention". "Biologically pure culture" is understood herein to mean that culture in which the microorganism or the strain of the invention is in a proportion equal to or greater than 99% with respect to other possible microorganisms present in the culture, and which can contain metabolites derived from the strain of the invention.

As understood by a person skilled in the art, the strain of the invention or the biologically pure culture of the invention can be comprised in a biofertiliser or biostimulant composition for plant development and growth. In that sense, another aspect of the present invention relates to a composition comprising the strain of the invention or the biologically pure culture of the invention, hereinafter the "composition of the invention".

In another particular embodiment of the present invention, the composition of the invention comprises the strain of the invention in the form of microsclerotia, preferably the strain of the invention is in the form of solution, and more preferably at a concentration of between 1·10² and 1·10⁴ microsclerotia per mL of solution. By considering in the present invention that a Colony Forming Unit (CFU) is equivalent to a microsclerotium, in another particular embodiment the composition of the invention comprises a concentration of the strain of the invention of between 1·10² and 1·10⁴ CFU/mL of solution. As understood by a person skilled in the art, the strain of the invention can be comprised in any solution of the state of the art viable in cell culture. Examples of solutions of the strain of the invention include, without limitation, water and salts such as KCI or NaCl.

As understood by a person skilled in the art, the composition of the invention may further comprise at least one co-formulant which facilitates and aids in plant growth along with the strain of the invention. The term "co-formulant" refers herein to a second composition, solution, mixture, element, or product which is not the strain of the invention or the biologically pure culture of the invention and which is widely used in agriculture or gardening to facilitate, promote, and aid in plant growth, to improve the quality or fertility of agricultural cultivation soil, as well as to improve its physicochemical characteristics, to improve the quality of the composition of the invention and uses thereof as biofertiliser, or to improve the quality of the harvests.

Therefore, in another particular embodiment of the invention, the composition of the invention further comprises at least one co-formulant.

Identifying the types of agricultural co-formulants in the state of the art is a routine practice for a person skilled in the art. Examples of agricultural co-formulants include, but are not limited to, adjuvants, growth-stimulating agent, or biological control agent. Therefore, in another particular embodiment of the composition of the present invention, the co-formulant is selected from the list consisting of: adjuvant, growth-stimulating agent, biological control agent, and any combination thereof.

The term "adjuvant" refers herein to a substance, compound, or product which is incorporated in the composition of the invention to improve the performance of the biofertilizers or agrochemicals through increasing contact area, retention and absorption, correcting application water problems, reconciling and stabilising product mixture. Examples of adjuvants include, but are not limited to, emulsifier or surfactant, surface active agents, dispersing agents, pH regulators, minerals, salts, acids, stabilisers, and essences.

In another particular embodiment of the present invention, the adjuvant of the composition of the invention is selected from the list consisting of: emulsifier or surfactant, surface active agents, dispersing agents, pH regulators, minerals, salts, acids, stabilisers, essences, and any combination thereof.

The term "growth-stimulating agent" refers herein to a substance, compound, or product which facilitates, improves, and promotes plant growth and development along with the strain of the invention. Examples of plant growth-stimulating agents include, but are not limited to, nitrogen, phosphorus, and/or potassium fertilizers and combinations of two or three thereof, polysorbates associated with fatty acids, polysorbates, asparagine, mannitol, organic acids, macroelement- or microelement-chelating phytohormones (for example, auxins, gibberellins, or cytokinins), and algae or extracts thereof.

The term "biological control agent" refers herein to an agent, natural medium, living being, or microorganism, of natural origin, which control, eliminate, reduce, stabilise pests or diseases caused by pathogens in agricultural plants, harvests, or crops. Examples of biological control agents include, but are not limited to, phytohormones, organic compounds such as alcohols or acetones, essential oils, or pherohormones secreted by plants, trap crops, competing crops, predatory organisms (for example, coccinellids, syrphid flies, mites, insects, or nematodes), parasitic organisms and pathogenic organisms or biological pesticides (for example, bacteria such as *Bacillus thuringiensis* or *Paenibacillus popilliae,* entomopathogenic fungi, or viruses such as *Baculovirus*)*.*

As understood by a person skilled in the art, this composition may further comprise at least a second strain other than *Rutstroemia calopus* deposited in the Spanish Type Culture Collection (CECT) with accession number CECT 21150.

As understood by a person skilled in the art, for the use or application of the composition of the invention as biofertilizer in plants and crops, said composition can be in solid, liquid, or gaseous state. In a particular embodiment of the present invention, the composition of the invention is in solid, liquid, gel, or colloidal state.

As understood by a person skilled in the art, in order to promote plant growth and development, there is a need to facilitate the interaction of the agricultural plants or crops with the strain of the invention or with the biologically pure culture of the invention, or alternatively facilitate the anchoring of the plant or seeds. To that end, fixing the composition of the invention in suitable agricultural supports is advantageous.

In that sense, in another preferred embodiment, the composition of the invention further comprises an agricultural support or agriculturally acceptable support.

The terms "agricultural support" or "agriculturally acceptable support" used interchangeably herein refers to a natural or synthetic element, compound, mixture, product, or solution, mineral or organic, which, in pure form or in mixture, allows the anchoring of the root system of the plant, therefore playing a role for supporting and holding the plant or seeds, as well as also facilitating the interaction of the agricultural plants or crops with the composition of the invention.

Identifying the type of agriculturally acceptable supports in the state of the art both in soil cultivation and in hydroponic cultivation is a routine practice for a person skilled in the art. Examples of agricultural supports for soil cultivation include, but are not limited to, organic or inorganic substrates. Examples of agricultural supports for hydroponic cultivation include, but are not limited to, water or saline solution. The agricultural supports, both for soil cultivation and for hydroponic cultivation, can be in the form of powder, granule, wettable powders, aqueous or non-aqueous media, suspensions, or dispersions. Likewise, the agricultural support can be a biopolymer matrix. Examples of biopolymer matrix include, but are not limited to, calcium alginate and agar.

In that sense, in another particular embodiment of the invention, the agricultural support of the composition of the invention is selected from the list consisting of: organic substrate, inorganic substrate, water, saline solution, or any combination thereof.

As understood by a person skilled in the art, any agriculturally acceptable organic or inorganic substrate is valid for use in the present invention. Examples of agriculturally acceptable organic substrates include, but are not limited to, clay, plant waste, cork powder, cellulose, peat, coconut fiber, or compost. Therefore, in another particular embodiment of the agricultural support of the composition of the invention, the agriculturally acceptable organic substrate is selected from the list consisting of: clay, perlite, vermiculite, plant waste, cork powder, cellulose, peat (Sphagnum peat), coconut fibre, compost, and any combination thereof.

In another particular embodiment, the agricultural support is compost obtained from plant materials which are selected from the list consisting of: grapevine pomace, mushroom, horticulture waste, municipal solid waste, gardening waste, vermicompost, and combinations thereof.

As is known to a person skilled in the art, the composition of the invention may further comprise a seed. In that sense, another particular embodiment relates to the composition of the invention further comprising a seed.

The term "seed" refers herein to a seed, kernel or pip and progeny thereof responsible for giving rise to a plant of any species, particularly plants of agronomic, forestry, food (for human or animal consumption), ornamental, or energy interest.

The present invention describes the use of *Rutstroemia calopus* CG11560 (deposit number CECT 21150), the strain of the invention, as fertilizer or as plant growth promoting agent in plants, that is able to promote efficient plant growth, even under conditions of water and salt stress.

To that end, another aspect of the present invention is the use of the *Rutstroemia calopus* strain deposited in the Spanish Type Culture Collection (CECT) with accession number CECT 21150 (the strain of the invention), as well as of the biologically pure culture of the invention and of the composition of the invention, as fertilizer or to enhance plant growth or development, hereinafter "the use of the invention".

The terms "biofertilizer", "fertilizer", or "biostimulant", used interchangeably in the present invention, refers to an agriculturally acceptable product comprising living microorganisms, specifically the *Rutstroemia calopus* strain of the present invention which, when applied to plants, harvests, crops, or seeds promotes, facilitates, improves, increases, enhances plant development or growth, i.e., promotes the growth or development of the root system of plants, the elongation of stems, flowering, and/or the development of fruits and seeds, aiding in improving the yield and quality of agricultural crops, and furthermore providing protection against pathogens.

The strain of the invention, *Rutstroemia calopus* CG11560 with deposit number CECT 21150, the biologically pure culture of the invention, and the composition of the invention, as well as the term "enhancing plant growth" have already been described in the preceding paragraphs of the present document and apply equally to this inventive aspect, as well as to all the particular embodiments thereof.

The term "fertilizer" refers herein to a compound, element, composition, mixture, product, or solution comprising microorganisms such as bacteria and/or fungi, providing the plants with the nutrients needed for their development, while improving soil quality at the same time, aiding in achieving an optimized and natural microbiological environment and in improving agricultural production without harming the environment.

As understood by a person skilled in the art, any plant species can be used to stimulate growth, particularly growth in early stages. Examples of plants include, but are not limited to, plants of agronomic or cultivation interest (for example, plants of interest for human or animal consumption or plants of forestry interest) and plants of ornamental interest.

In a preferred embodiment, the plant of the use of the invention is a crop plant or an ornamental plant.

Examples of plants of agronomic interest include, but are not limited to, oats, barley, corn, wheat, olive tree, grapevine, almond tree, soybean, spruce, oak, pine tree, rye, beet, potato, carrot, garlic, rice, onion, tomatoes, sunflower, pepper, melon, strawberry, beans, orange, apple, peach, lemon, and pear. Examples of plants of ornamental interest include, but are not limited to, grass, sorghum, rosebush, geranium, gladiolus, paniculata, carnation, hyacinth, petunia, cacti and succulents.

As described above, said enhancing plant growth also furthermore occurs under conditions of water or salt stress when the strain of the invention is applied. To that end, a particular embodiment of the use of the invention relates to the use of the strain of the invention, the biologically pure culture, or the composition of the invention, wherein the growth or development of the plant occurs under conditions of water or salt stress.

As described in the present invention, applying the *Rutstroemia calopus* CG11560 strain (deposit number 21150) in different cultures promotes and enhances plant growth and development of said crops, even when the crops are under conditions of water and salt stress, thereby describing a biostimulation method for plant growth.

Therefore, another aspect of the present invention relates to a method for enhancing or promoting plant or crop growth, hereinafter the "method of the invention", which consists of:
(i) contacting a plant or the seed of a plant with a fertilizer which is selected from the list consisting of the strain of the invention, the biologically pure culture of the invention, and the composition of the invention; and
(ii) developing the seed or the plant of step (i).

The strain of the invention, *Rutstroemia calopus* CG11560 with deposit number CECT 21150, the biologically pure culture of the invention, and the composition of the invention, have already been described in the preceding paragraphs of the present document and apply equally to this inventive aspect, as well as to all the particular embodiments thereof. Likewise, the term "enhancing plant growth" has been described above in the present document and applies equally to this inventive aspect.

Alternatively, step (i) of the method of the invention refers to sowing a seed comprised in the composition of the invention (described above in a particular embodiment of the present invention), and then developing said seed.

As understood by a person skilled in the art, the strain of the invention, the biologically pure culture of the invention, or the composition of the invention can be applied to the plants or seeds in liquid or solid form in soil cultivation or in liquid form in hydroponic cultivation. In that sense, in a particular embodiment of the method of the invention, the strain of the invention, the biologically pure culture of the invention, or the biofertilizer composition of the invention are applied in liquid or solid form in soil cultivation or in a hydroponic manner.

The application of the fertilizer in the method of the invention, i.e., of the strain, the biologically pure culture, or the composition of the invention, can be carried out such that the seed, plant, or parts of a plant can be coated completely or partially. This coating can be carried out by means of conventional processes including, but are not limited to, dipping or pulverising the seed, plant, or its parts with the fertilizer.

Also as understood by a person skilled in the art, the strain of the invention, the biologically pure culture of the invention, or the composition of the invention can be applied in a radicular manner (roots) by means of conventional methods known in the state of the art such as substrate irrigation.

As understood by a person skilled in the art, any plant species can be used to stimulate growth, particularly growth in early stages. Examples of plants of agronomic or cultivation interest and of ornamental interest have been described above and apply equally to this aspect of the invention.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Figure 1****.** Mycelial growth *in vitro* of *Rutstroemia calopus* CG11560 at different salt concentrations (0, 1, 2, 5, 10, 15, 20 g·L⁻¹) and temperatures (25°C and 35°C).
**Figure 2****.** Effect of the application of *Rutstroemia calopus* CG11560 on different morphological parameters of melon seedlings. A) Aerial dry weight of melon seedlings (g) and B) Root dry weight of melon seedlings (g). The asterisk indicates statistically significant difference between treatments.
**Figure 3****.** Plant growth of melon seedlings subjected to salt stress (0.5 g/L, 1 g/L, 1.5 g/L, and 2 g/L of salt) after the application of *Rutstroemia calopus* CG11560. The asterisk indicates statistically significant difference between treatments.
**Figure 4****.** Plant growth of pepper seedlings after the application of *Rutstroemia calopus* CG11560. The asterisk indicates statistically significant difference between treatments.
**Figure** 5. Plant growth of cucumber seedlings after the application of *Rutstroemia calopus* CG11560 under conditions of salt stress (I). The letters a and b indicate statistically significant differences between treatments.
**Figure 6****.** Plant growth of cucumber seedlings after the application of *Rutstroemia calopus* CG11560 under conditions of salt stress (II). The letters a and b indicate statistically significant differences between treatments.
**Figure 7****.** Plant growth of cucumber seedlings after the application of *Rutstroemia calopus* CG11560 under conditions in which fertilization is reduced to 50%. The letters a and b indicate statistically significant differences between treatments.
**Figure 8****.** Plant growth of cucumber seedlings after the application of *Rutstroemia calopus* CG11560 under conditions in which fertilization is reduced to 80%. The letters a and b indicate statistically significant differences between treatments.

### EXAMPLES

Next, the invention will be illustrated by means of tests performed by the inventors that demonstrate the effectiveness of the product of the invention.

### Materials and Methods

The inventors isolated the strain from the genus *Rutstroemia, Rutstroemia calopus* CG11560, from the roots of wild plants in the coast of Almería (Spain).

Disinfected roots were sown in a petri dish with PDA medium and a subsequent identification of the formed colonies was performed. The resulting pure strain was named CG11560, identified by means of PCR (polymerase chain reaction), and the ITS (internal transcriber spacer) region of ribosomal DNA (rDNA), belonging to the *Rutstroemia calopus* species, was subsequently sequenced. It has been identified by means of sequencing the ITS (internal transcriber spacer) region of rDNA using the primers ITS-1 (SEQ ID NO: 1) and ITS-4, 5'(TCCTCCGCTTATTGATATGC) (SEQ ID NO: 4). The sequence obtained with the ITS1 primer, which is a DNA fragment of the ribosomal gene of CG11560 is a non-coding sequence, and which is identified as SEQ ID NO 1.

The strain was grown in a PDA (potato dextrose agar) agar culture medium at room temperature and the amount of conidia produced per petri dish at different salt concentrations (0, 1, 2, 5, 10, 15, 20 g·L⁻¹) was evaluated.

To determine the promoting effect of *Rutstroemia calopus* CG11560, melon seeds of the "Piñonet piel de sapo" variety were disinfected and pregerminated; they were planted in 300 mL pots in commercial peat. One seed was placed per pot. Simultaneously with sowing, 5 mL of water (T0) or 5 mL of microsclerotia suspension were applied per pot at a concentration of 10²·mL⁻¹. The test was carried out under greenhouse conditions, i.e., under temperature and humidity conditions that are not constant or controlled (passive conditions in which the temperature varies from 20 to 35°C and humidity varies from 70% to 90%). Each treatment consisted of 25 repetitions. The plants were fertilized daily with a commercial fertilizer (1 g/l of Nitrofoska 13(N) + 9(P2O5) + 16(K2O) [+4 (MgO) + 17(SO3)).

To determine the promoting effect of *Rutstroemia calopus* CG11560 in salt conditions, melon seeds of the Piñonet piel de sapo variety were disinfected and pregerminated and planted in 300 mL pots in a commercial peat. One seed was placed per pot. Simultaneously with sowing, 5 mL of water (T0) or 5 mL of microsclerotia suspension were applied per pot at a concentration of 10²·mL⁻¹. The test was carried out under greenhouse conditions. Each treatment consisted of 25 repetitions. The plants were fertilised daily with a commercial fertiliser (1 g/l of Nitrofoska 13(N) + 9(P2O5) + 16(K2O) [+4 (MgO) + 17(SO3)). When the first true leaf was expanded, the plants were watered with the different salt concentrations, (0, 0.5, 1, 1.5, and 2 gL⁻¹) corresponding to an electrical conductivity of 4.00, 4.31, 5.30, 6.39, and 7.16 dS/m. The plants were analyzed after 45 days.

To determine the effect of *Rutstroemia calopus* CG11560 on the development of pepper seedlings under seedbed conditions, *Rutstroemia calopus* CG11560 was applied at a dose of 10² microsclerotia per mL of water. The application was performed by substrate irrigation (R). For each plant, 1 mL of the suspension was added in a single application. The duration of the test was 40 days, and data on some morphological parameters was then collected before sowing in the soil.

To determine the effect of *Rutstroemia calopus* CG11560 on the development of cucumber seedlings under salt conditions, the seedlings were sown in pots with a volume of 1 l and the substrate used was peat. Four discs of 0.5 cm in diameter containing the mycelium of *Rutstroemia calopus* CG11560 were applied on the substrate. Irrigations were performed with a 2 g/L saline solution corresponding to an electrical conductivity of about 7 dS/m. The duration of the test was 60 days, and data on some morphological parameters was then collected.

To determine the effect of *Rutstroemia calopus* CG11560 on the development of cucumber seedlings under conditions in which fertilization is reduced to 50%, the seedlings were sown in pots with a volume of 1 L and the substrate used was peat. Four discs of 0.5 cm in diameter containing the mycelium of *Rutstroemia calopus* CG11560 were applied on the substrate. Irrigations were performed with a solution containing a 50% reduction in fertilization with respect to standard fertilization (1 g/l of commercial fertilizer NPK 15-15-15). The duration of the test was 60 days, and data on morphological parameters was then collected.

To determine the effect of *Rutstroemia calopus* CG11560 on the development of cucumber seedlings under conditions in which fertilization is reduced to 80%, the seedlings were sown in pots with a volume of 1 L and the substrate used was peat. Four discs of 0.5 cm in diameter containing the mycelium of *Rutstroemia calopus* CG11560 were applied on the substrate. Irrigations were performed with a solution containing a 80% reduction in fertilization with respect to standard fertilization (1 g/L of fertilizer). The duration of the test was 60 days, and data on morphological parameters was then collected.

### Results

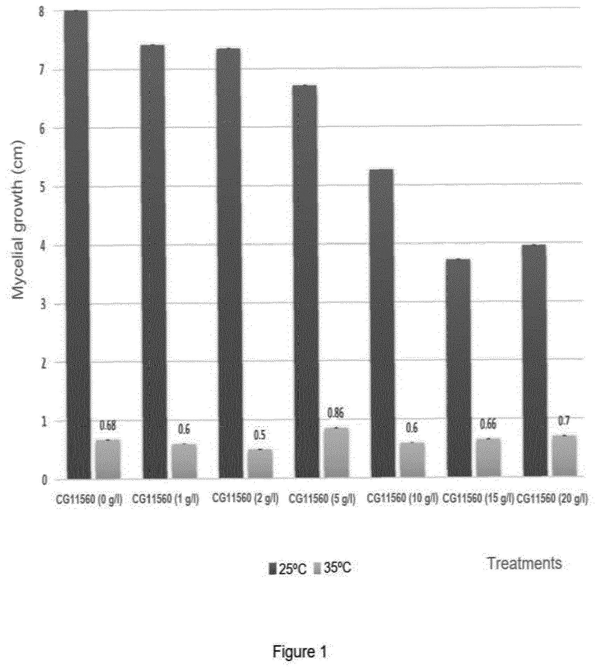

Figure 1 shows the results of mycelial growth of *Rutstroemia calopus* CG11560 at different salt concentrations and temperatures. Spore assessment could not be performed because it does not experience sporulation in this agar medium. It only produces mycelium and microsclerotia in large quantities. Inhibition of mycelial growth occurs at the different salt concentrations tested at 25°C, representing a 50% reduction at 20 gL⁻¹. A drastic reduction in growth is observed at 35°C. Therefore, growth is affected by both salt concentration and temperature.

Figure 2 shows the results obtained after the application of *Rutstroemia calopus* CG11560 in the development of melon seedlings. The results reveal that there is an increase in the morphological parameters evaluated after the application of *Rutstroemia calopus* CG11560. An increase of 27.08% and 64.69% is observed in the dry weight of the aerial and root parts, respectively.

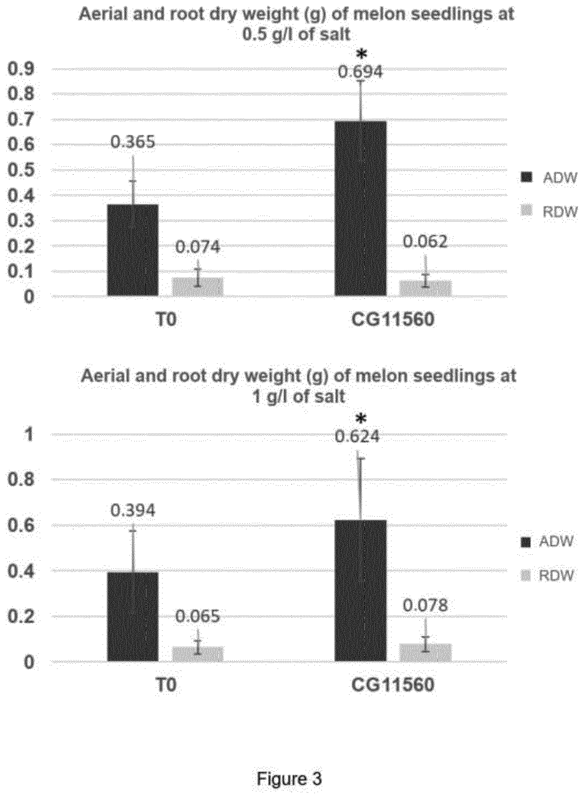

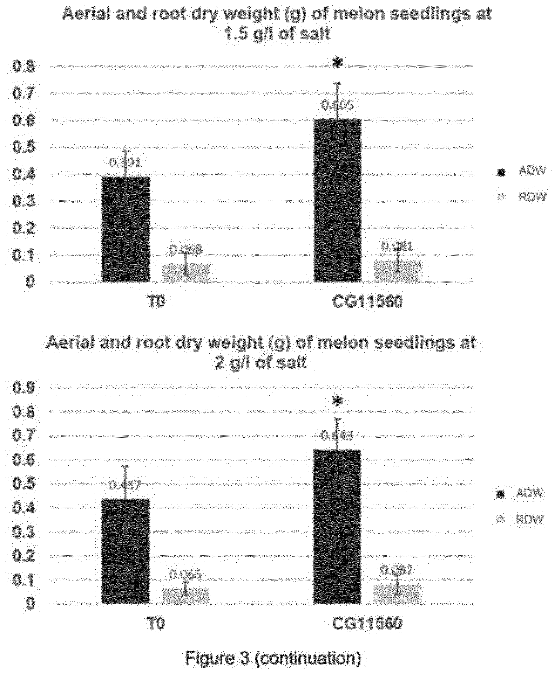

Figure 3 shows the results obtained after the application of *Rutstroemia calopus* CG11560 in the development of melon seedlings subjected to salt stress. The incorporation of *Rutstroemia calopus* CG11560 on the substrate subjected to high electrical conductivities softens the effect of saline stress, not causing a decrease in the dry weight of the aerial or root part, except at a concentration of 0.5 g/L that causes a decrease in root dry weight, although without significant differences. An increase of 90.13, 58.37, 54.73, and 47.13% is observed in the dry weight of the aerial part at 0.5, 1, 1.5, and 2 g·L⁻¹, respectively. In the case of the root, there are variations in the percentage of dry weight of -16.21, 20, 19.11, and 26.15% at 0.5, 1, 1.5, and 2 gL⁻¹, respectively.

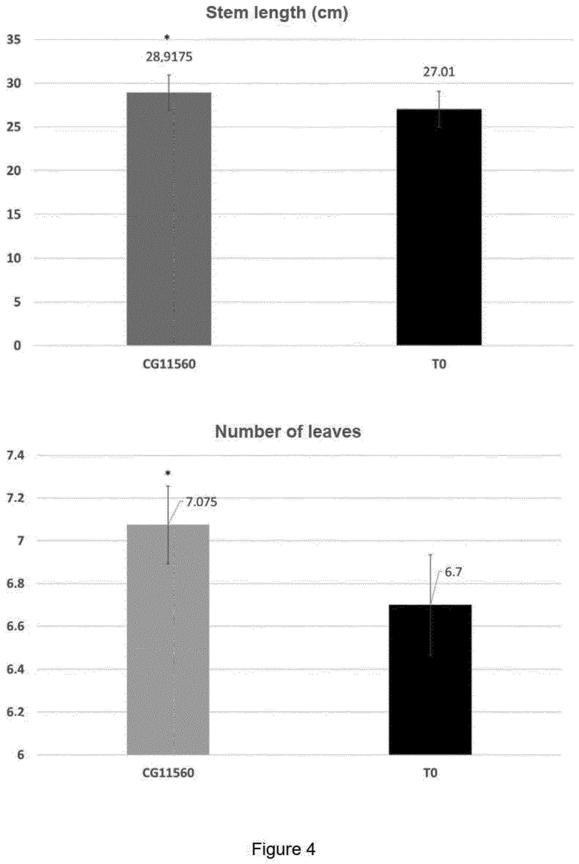

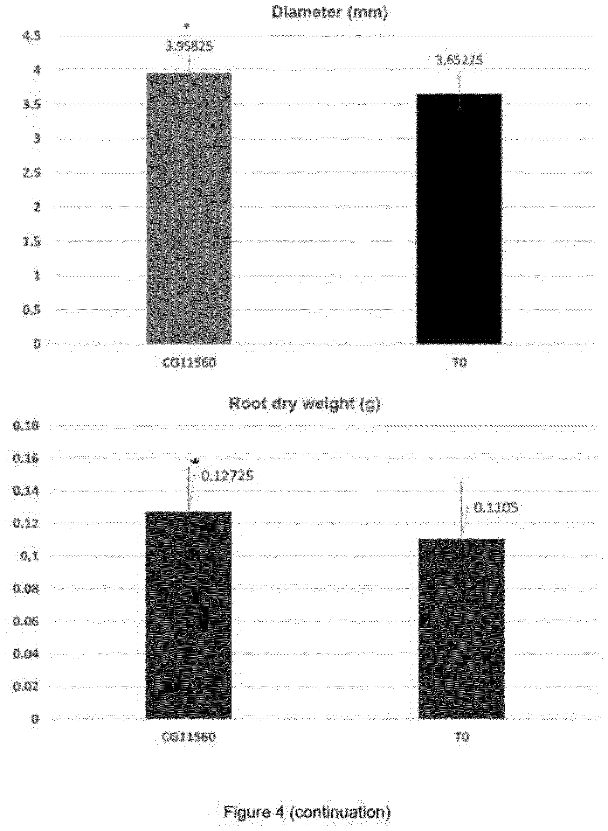

Figure 4 shows the results obtained after the application of *Rutstroemia calopus* CG11560 in the development of pepper seedlings under seedbed conditions. The results reveal that there is an increase of 7% in the height of the pepper seedlings, 8.2% in diameter, 5.5% in the number of leaves, and 15.45% after the application of *Rutstroemia calopus* CG11560.

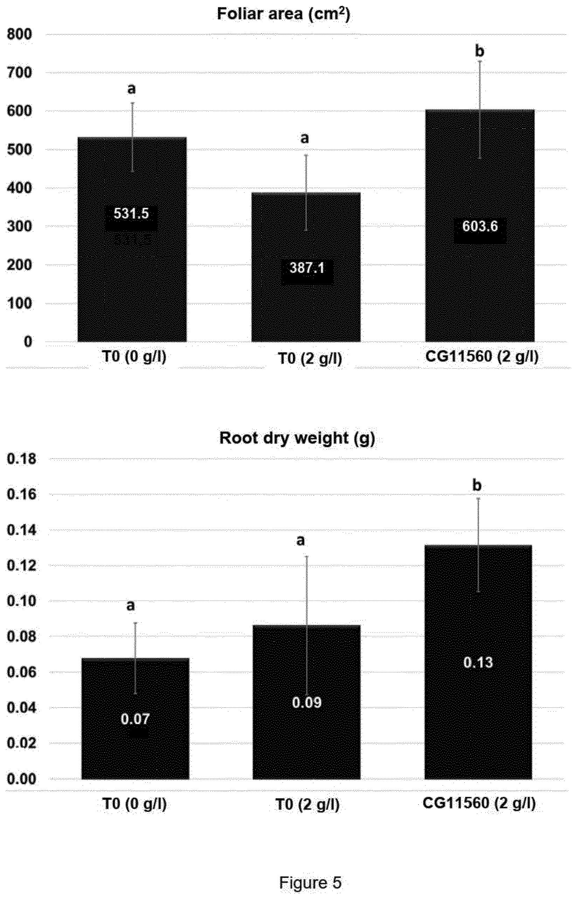

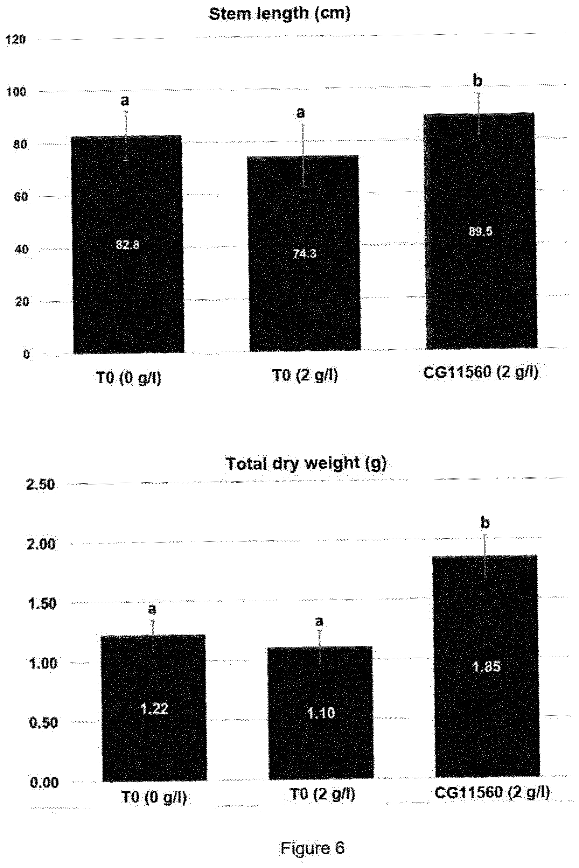

Figures 5 and 6 show the results obtained after the application of *Rutstroemia calopus* CG11560 in the development of cucumber seedlings under salt conditions. The results reveal that there is an increase of 56% in the foliar area and 44.4% in the root dry weight compared to the control under salt conditions after the application of *Rutstroemia calopus* CG11560 (Figure 5). Likewise, there is an increase of 13.5% in the foliar area and 85.1% in the root dry weight when compared to the control at 0 g/L of salt. Likewise, there is an increase of 20.45% in stem length and 68.18% in the total dry weight compared to the control under salt conditions after the application of *Rutstroemia calopus* CG11560 (Figure 6). Likewise, there is an increase of 8% in stem length and 51.63% in the total dry weight when compared to the control at 0 g/L of salt.

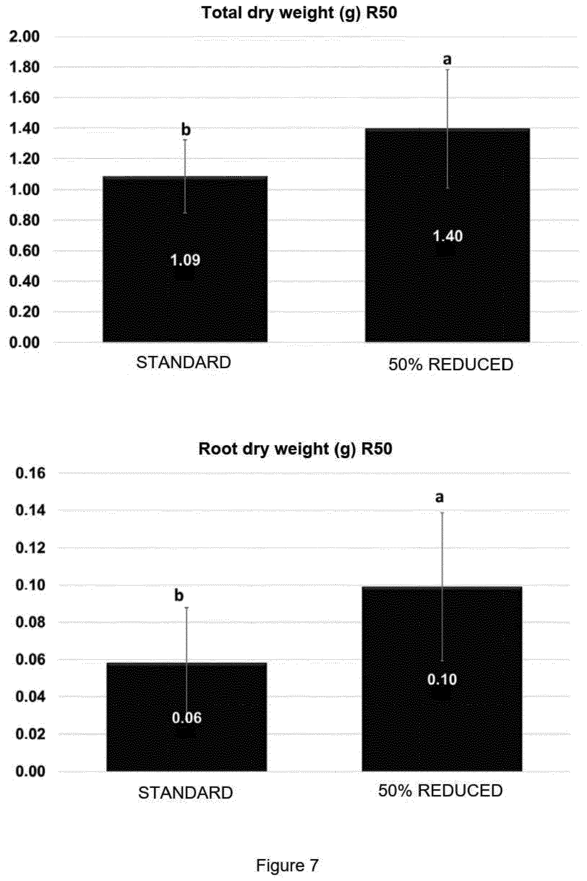

Figure 7 shows the results obtained after the application of *Rutstroemia calopus* CG11560 in the development of cucumber seedlings under the conditions in which fertilization is reduced to 50%. The results reveal that there is an increase of 28.44% in the total weight of the plant after the application of *Rutstroemia calopus* CG11560 when fertilization is reduced. Likewise, there is an increase of 66.66% of the root dry weight when compared to standard fertilization.

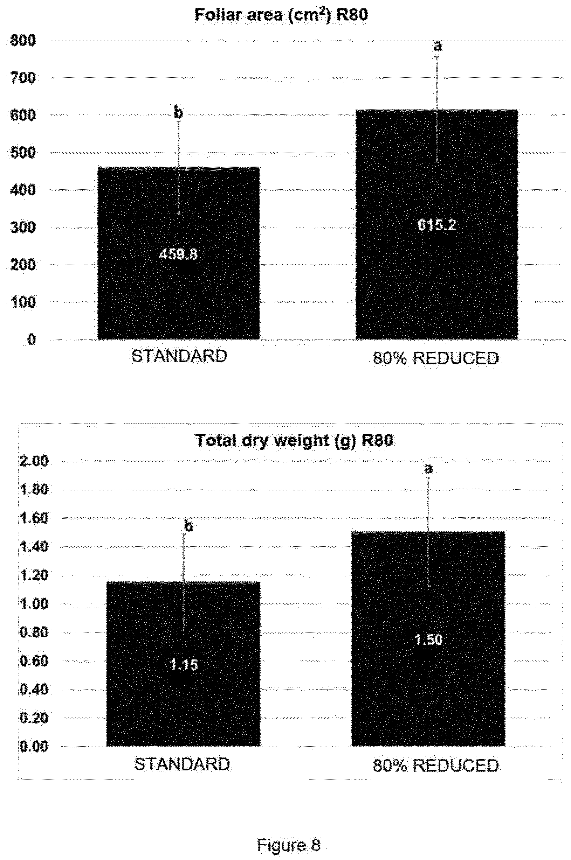

Figure 8 shows the results obtained after the application of *Rutstroemia calopus* CG11560 in the development of cucumber seedlings under the conditions in which fertilization is reduced to 80%. The results reveal that there is an increase of 33.8% in the foliar area of the plant after the application of *Rutstroemia calopus* CG11560 when fertilization is reduced. Likewise, there is an increase of 30.43% of the total dry weight when compared to standard fertilization.

## Claims

1. A strain of the species *Rutstroemia calopus* deposited in the Spanish Type Culture Collection (CECT) with accession number CECT 21150.

2. The strain according to claim 1, wherein the strain is in the form of viable cells.

3. The strain according to claim 2, wherein the viable cells are in the form of microsclerotia.

4. A biologically pure culture of the strain according to any one of claims 1 to 3.

5. A composition comprising the strain according to any one of claims 1 to 3 or the biologically pure culture according to claim 4.

6. The composition according to claim 5, wherein the strain is in the form of microsclerotia at a concentration between 1·10² and 1·10⁴ CFU per mL of solution.

7. The composition according to any of claims 5 or 6, further comprising at least one co-formulant.

8. The composition according to claim 7, wherein the co-formulant is selected from the list consisting of: adjuvant, growth-stimulating agent, biological control agent, and any combination thereof.

9. The composition according to claim 8, wherein the adjuvant is selected from the list consisting of: emulsifier or surfactant, surface active agents, dispersing agents, pH regulators, minerals, salts, acids, stabilisers, essences, and any combination thereof.

10. The composition according to any one of claims 5 to 9, wherein the composition is in solid, liquid, gel, or colloidal state.

11. The composition according to any one of claims 5 to 10, wherein the composition further comprises an agricultural support.

12. The composition according to claim 11, wherein the support is selected from the list consisting of: organic substrate, inorganic substrate, water, saline solution, and any combination thereof.

13. The composition according to claim 12, wherein the organic substrate is selected from the list consisting of clay, plant waste, cork powder, cellulose, peat, coconut fibre, compost, and any of the combinations thereof.

14. The composition according to any one of claims 5 to 13, wherein the composition further comprises a seed.

15. Use of the strain according to any one of claims 1 to 3, of the biologically pure culture according to claim 4, or of the composition according to any one of claims 5 to 13 as fertilizer or to enhance or promote the growth of a plant.

16. Use according to claim 15, wherein the plant is a crop plant or an ornamental plant.

17. Use according to any one of claims 15 or 16, wherein the growth of the plant occurs under conditions of water or salt stress.

18. A method for enhancing or promoting the growth of plants which comprises:
(i) contacting a plant or the seed of a plant with a fertilizer which is selected from the list consisting of the strain described in any one of claims 1 to 3, the biologically pure culture according to claim 4, and the composition according to any one of claims 5 to 14; and
(ii) developing the seed or the plant of step (i).

19. The method according to claim 18, wherein the contact in step (i) is carried out by means of applying the fertilizer in liquid form, solid form, or in a hydroponic manner.
